# EUROPÄISCHE PATENTSCHRIFT

(11) **EP 2 670 455 B1**
(45) Veröffentlichungstag und Bekanntmachung des Hinweises auf die Patenterteilung: **04.10.2017**
(21) Anmeldenummer: 12703442.9
(22) Anmeldetag: 27.01.2012
(51) Int. Cl.: A61M 1/36, A61M 5/145, A61M 1/16, A61M 5/14

(54) **KLEMMHALTERUNG FÜR EINE SPRITZE EINER DOSIERVORRICHTUNG, DOSIERVORRICHTUNG UND BLUTBEHANDLUNGSVORRICHTUNG**
CLAMP MOUNTING FOR A SYRINGE OF A DOSING DEVICE, DOSING DEVICE AND BLOOD TREATMENT DEVICE
DISPOSITIF DE RETENUE PAR SERRAGE POUR UNE SERINGUE DE DISPOSITIF DE DOSAGE, DISPOSITIF DE DOSAGE ET DISPOSITIF DE TRAITEMENT DE SANG

(30) Priorität: 31.01.2011 DE 102011009908; 31.01.2011 US 201161437690 P
(43) Veröffentlichungstag der Anmeldung: 11.12.2013
(73) Patentinhaber: Fresenius Medical Care Deutschland GmbH, 61352 Bad Homburg (DE)
(72) Erfinder: OESTERREICH, Stefan, 61267 Neu-Anspach (DE); STEJSKAL, Martin, 65812 Bad Soden (DE)
(74) Vertreter: Bobbert & Partner Patentanwälte PartmbB
(86) Internationale Anmeldenummer: PCT/EP2012/000367
(87) Internationale Veröffentlichungsnummer: WO 2012/104042

(56) Entgegenhaltungen:
- WO-A1-97/02056
- WO-A1-2007/012915
- US-A- 5 545 140
- US-B1- 6 808 149

## Beschreibung

Die vorliegende Erfindung betrifft eine Klemmhalterung gemäß Anspruch 1. Sie betrifft ferner eine Dosiervorrichtung gemäß Anspruch 10 und eine Blutbehandlungsvorrichtung gemäß Anspruch 12.

Aus der medizinischen Praxis sind Dosiervorrichtungen bekannt. In Dosiervorrichtungen dieser Art werden eine Spritze oder eine andere Medikamentenabgabeeinrichtung eingelegt, welche ein medizinisches flüssiges Fluid - z. B. während einer Blutbehandlung wie der Hämodialyse extrakorporal zugegebenes Heparin - enthalten. Mittels der Dosiervorrichtungen wird das Fluid nach Bedarf oder Einstellung abgegeben.

WO 2007/012915 A1 offenbart eine Klemmhalterung mit einem verschwenkbaren Haken, US 5 545 140 A offenbart eine Klemmhalterung mit einer Klemmvorrichtung, WO 97/02056 A1 offenbart eine Halterung für einen Behälter und US 6 808 149 B1 offenbart eine Halterung für Flaschenhälse.

Eine Aufgabe der vorliegenden Erfindung ist es, eine weitere Klemmhalterung der oben genannten Art vorzuschlagen.

Die erfindungsgemäße Aufgabe wird durch eine Klemmhalterung mit den Merkmalen des Anspruchs 1 gelöst.

Die erfindungsgemäße Klemmhalterung ist vorgesehen zur Aufnahme wenigstens eines Abschnitts einer Medikamentenabgabeeinrichtung innerhalb einer medizinischen Dosiervorrichtung (im Folgenden auch kurz: Dosiervorrichtung) oder zur Begrenzung des Abschnitts in wenigstens einer Bewegungsrichtung. Die Medikamentenabgabeeinrichtung weist eine Aufnahmekammer für eine abzugebende Medikamentenlösung oder Substanz auf. Die Klemmhalterung weist wenigstens eine Klemmvorrichtung auf, welche vorgesehen ist zum Halten, Begrenzen, Aufnehmen des Abschnitts, zum Beschränken einer Bewegungsfreiheit des Abschnitts - oder eines Teils hiervon - der Medikamentenabgabeeinrichtung und/oder zum Verschieben des Abschnitts. Die Klemmvorrichtung weist wenigstens eine Vertiefung auf, welche vorgesehen ist zur Aufnahme des Abschnitts - oder eines Teils hiervon - der Medikamentenabgabeeinrichtung oder zu seiner Begrenzung.

Die Vertiefung weist eine Abgrenzung gegenüber anderen oder zum Abschnitt benachbarten Abschnitten der Klemmvorrichtung oder eine Kante auf oder stellt eine solche jeweils dar. Die Vertiefung, die Abgrenzung und/oder die Kante oder jeweils ein Teilbereich hiervon verlaufen bogenförmig, kurvenförmig, gekrümmt oder dergleichen.

Die erfindungsgemäße Aufgabe wird auch durch eine Dosiervorrichtung mit den Merkmalen des Anspruchs 10 gelöst. Diese weist wenigstens eine erfindungsgemäße Klemmhalterung auf.

Die erfindungsgemäße Aufgabe wird auch durch eine Blutbehandlungsvorrichtung mit den Merkmalen des Anspruchs 12 gelöst. Diese weist wenigstens eine erfindungsgemäße Klemmhalterung oder wenigstens eine erfindungsgemäße Dosiervorrichtung auf.

Vorteilhafte Weiterentwicklungen und spezielle Ausgestaltungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll bestimmte erfindungsgemäße Ausführungsformen erläutern.

Der bogenförmig oder kurvenförmig gestaltete Verlauf der Vertiefung, der Abgrenzung, der Kante oder des Teilbereichs ist in bestimmten erfindungsgemäßen Ausführungsformen jeder Verlauf, welcher insgesamt oder über wenigstens einen Bereich hiervon anders als gerade verläuft, also nicht gerade verläuft.

Die Vertiefung in der Klemmvorrichtung der erfindungsgemäßen Klemmhalterung dient der Beschränkung der Bewegungsfreiheit eines Abschnitts der Medikamentenabgabeeinrichtung. So kann mittels der Vertiefung beispielsweise eine axiale Verschiebbarkeit des Abschnitts beschränkt sein. Z. B. kann die Klemmvorrichtung eine unerwünschte axiale Verschiebbarkeit eines Spritzenstempels verhindern. Zu diesem Zweck wird beim Verklemmen der Medikamentenabgabeeinrichtung in der Klemmvorrichtung der besagte Abschnitt in die Vertiefung eingeführt und/oder von dieser begrenzt und kann nach seinem Einführen oder Unterschieben nur noch gemeinsam mit der Klemmvorrichtung in seiner axialen Richtung verschoben werden. Erfindungsgemäß ist die Vertiefung als Nut ausgestaltet. Diese besondere Ausgestaltung der Vertiefung hat den Vorteil, dass ein unerwünschtes Verschieben des besagten Abschnitts in beide axiale Richtungen beschränkt sein kann (z. B. sowohl vor als auch zurück). Zudem kann ein Führen oder aktives Bewegen des Abschnitts in beide axiale Richtungen mittels einer Nut möglich sein. Eine Nut kann auf einfache Weise eine spielfreie Beschränkung oder Begrenzung sicherstellen.

In einigen erfindungsgemäßen Ausführungsformen ist die Vertiefung derart innerhalb oder an der Klemmvorrichtung vorgesehen, dass ein erster Querschnitt durch die Klemmvorrichtung oberhalb der Vertiefung dieselbe Form hat wie ein zweiter Querschnitt durch die Klemmvorrichtung unterhalb der Vertiefung. In diesen Ausführungsformen kann die Vertiefung somit eine Unterbrechung, nicht aber einen Abschluss der Klemmvorrichtung (z. B. zu ihrem Stirnende hin) darstellen.

Die Klemmvorrichtung ist in einigen erfindungsgemäßen Ausführungsformen als Klemmbacke ausgestaltet.

In manchen erfindungsgemäßen Ausführungsformen weist die Klemmvorrichtung wenigstens eine Feder auf oder ist mit einer solchen verbunden, welche die Klemmwirkung bewirkt oder zu dieser beiträgt.

In bestimmten erfindungsgemäßen Ausführungsformen weist die Klemmvorrichtung elastisches Material auf, besteht aus diesem oder ist mit solchem verbunden, um mittels der Elastizität des Materials die Klemmwirkung zu bewirken oder zu dieser beizutragen.

Die Vertiefung ist in einigen erfindungsgemäßen Ausführungsformen über eine gesamte Tiefe der Klemmvorrichtung ausgestaltet. In anderen erfindungsgemäßen Ausführungsformen ist die Vertiefung über nur einen Teil der Tiefe der Klemmvorrichtung ausgestaltet. Erfindungsgemäß ist die Vertiefung oder die Abgrenzung ganz oder in wenigstens einem Bereich hiervon kurvenförmig oder bogenförmig und weicht somit von einem geraden Verlauf ab. Die Abweichung von der Geraden bezieht sich in einigen erfindungsgemäßen Ausführungsformen - ausschließlich oder ergänzend - auf eine Ebene parallel zu einer Verschieberichtung des mittels der Klemmvorrichtung in seiner Bewegungsfreiheit begrenzten Abschnitts. Die Abweichung von der Geraden ist derart, dass der Verlauf der Abgrenzung in einer Ebene parallel zur Verschieberichtung des mittels der Klemmvorrichtung in seiner Bewegungsfreiheit begrenzten Abschnitts konkav zum Abschnitt (dieser kann z. B. als Spritzenstempel ausgestaltet sein) hin und konvex in einer Richtung von einer Abgabeöffnung für das Medikament ausgehend ist.

Der Abschnitt der Medikamentenabgabeeinrichtung kann ein relativ zur übrigen Medikamentenabgabeeinrichtung oder anderen Abschnitten hiervon bewegbar angeordneter Abschnitt sein. Er kann beispielsweise vorgesehen sein, um zum Zweck des Dosierens verschiebbar zu sein.

In bestimmten erfindungsgemäßen Ausführungsformen ist die Medikamentenabgabeeinrichtung eine Spritze, und der Abschnitt der Medikamentenabgabeeinrichtung ist ein in der Spritze zum Zwecke der Abgabe des Medikaments verschiebbarer Stempel.

In einigen erfindungsgemäßen Ausführungsformen weist die Klemmhalterung eine Auflage auf, auf welcher die Medikamentenabgabeeinrichtung in ihrem Gebrauchszustand und/oder bei der Medikamentenabgabe ruht. Sie kann dort insbesondere mit ihrem mittels der Klemmvorrichtung in seiner Bewegung beschränkten Abschnitt ruhen. Die Auflage weist einen Abrollradius auf, mittels oder unter welchem die Auflage an wenigstens einem Übergang einer Auflagefläche zu einer Stirnseite oder Seitenfläche abgerundet ist.

In bestimmten erfindungsgemäßen Ausführungsformen weist die Klemmvorrichtung wenigstens einen Stirnbereich oder eine Stirnfläche auf, welche(r) einen kurven- oder bogenförmigen Verlauf oder eine kurven- oder bogenförmig begrenzte Fläche aufweist.

In bestimmten erfindungsgemäßen Ausführungsformen weist die Klemmhalterung wenigstens oder genau zwei Klemmvorrichtungen zur Aufnahme des Abschnitts auf. Die Klemmvorrichtungen können insbesondere gleich oder identisch ausgestaltet sein; sie können allerdings auch unterschiedlich ausgestaltet sein (z. B. als linke oder rechte Klemmvorrichtung, oder anderweitig unterschiedlich).

In manchen erfindungsgemäßen Ausführungsformen ist eine Düse oder Öffnung der Medikamentenabgabeeinrichtung zum Ausbringen deren Inhalts mit einem Schraubgewinde ausgestaltet. Das Schraubgewinde kann vorgesehen sein, um einen Schlauch (z. B. Infusionsschlauch), eine Hohlnadel (Kanüle), einen Katheter, Dreiwegehähne oder Ähnliches hieran anzuschließen. Das Schraubgewinde kann als genormtes Verbindungssystem (insbesondere als "Luer-Lock") ausgestaltet sein.

In einigen erfindungsgemäßen Ausführungsformen ist die Dosiervorrichtung oder Medikamentenabgabeeinrichtung zum Fördern von Lösungen zur Antikoagulation vorgesehen, insbesondere zum Fördern von Citrat- oder Calciumlösungen, ferner Heparinlösungen.

In bestimmten erfindungsgemäßen Ausführungsformen ist die Blutbehandlungsvorrichtung eine Dialysevorrichtung, insbesondere eine Hämodialysevorrichtung, eine Hämofiltrationsvorrichtung, eine Hämodiafiltrationsvorrichtung oder dergleichen, insbesondere für eine Akutdialyse.

Bestimmte erfindungsgemäße Ausführungsformen weisen einen oder mehrere der im Folgenden genannten Vorteile auf.

In bestimmten erfindungsgemäßen Ausführungsformen ist ein "robustes" Abrüsten z. B. der Spritze aus der Dosiervorrichtung heraus oder von der Behandlungsvorrichtung herunter möglich, ohne dass hierdurch ein Schaden zu befürchten wäre. Unter einem robusten Abrüsten wird in diesem Zusammenhang ein Abrüsten verstanden, bei welchem nicht die an sich zu erwartende Vorsicht oder Sorgfalt aufgebracht wird. Letzteres beobachtet man vor allem bei einem Abrüsten, welches unter Zeitdruck erfolgt. Ein Verkanten z. B. des Spritzenstempels, bei welchem dieser in der Halterung des Griffstücks der Dosiervorrichtung hängenbleibt, wird in erfindungsgemäßer vorteilhafter Weise verhindert. Wenigstens kann die Wahrscheinlichkeit eines Verkantens verringert werden. Begünstigt wird dieser Vorteil dadurch, dass ein Verkippen des Abschnitts aufgrund der besonderen Geometrie der Vertiefung vorteilhaft erfolgen kann.

Erfindungsgemäß muss der Anwender nicht etwa die eine oder mehrere Klemmvorrichtungen separat lösen oder öffnen. Vielmehr erlaubt die besondere Geometrie der Klemmvorrichtung mit ihrer bogenförmigen Vertiefung ein selbsttätiges und sanftes Lösen der Klemmvorrichtung vom Abschnitt der Medikamentenabgabeeinrichtung bei dessen Herausziehen aus der Dosiervorrichtung.

Dieser vorteilhafte Effekt kann gesteigert werden durch den bogenförmigen Verlauf der Stirnfläche der Klemmvorrichtung.

Vorteilhafterweise ist die erfindungsgemäße Klemmhalterung geeignet, verschiedene Größen und Typen von Medikamentenabgabeeinrichtungen aufzunehmen. So können beispielsweise problemlos Spritzen mit 30 ml Volumen und Spritzen mit 50 ml Volumen verwendet werden. Sie alle können gleichermaßen problemlos auf die oben beschriebene robuste Art abgerüstet oder von der Dosiervorrichtung abgenommen werden. Dies ist nicht beschränkt auf Spritzen nur eines Herstellers. Tatsächlich können Produkte verschiedener Hersteller verwendet werden.

Die vorliegende Erfindung erlaubt einerseits eine einfaches Lösen z. B. einer Spritze von der Dosiervorrichtung. Andererseits ist die erfindungsgemäße Klemmhalterung in der Lage, z. B. den Stempel einer Spritze sicher zu halten und zu verhindern, dass sich dieser z. B. ungewollt vom Griffstück der Dosiervorrichtung lösen kann.

Die vorliegende Erfindung wird im Folgenden anhand der Figuren der beigefügten Zeichnung, in welchen identische Bezugszeichen gleiche oder ähnliche Bauteile bezeichnen, exemplarisch erläutert. In den Figuren gilt:
- **Fig. 1a**: zeigt eine erfindungsgemäße Dosiervorrichtung in einer Seitenansicht;
- **Fig. 1b**: zeigt die in Fig. 1a dargestellte Dosiervorrichtung in perspektivischer Ansicht;
- **Fig. 2a**: zeigt die Dosiervorrichtung der vorangegangenen Figuren in der Darstellung der Fig. 1a, bestückt mit einer Spritze in deren Gebrauchsanordnung;
- **Fig. 2b**: zeigt die Dosiervorrichtung der vorangegangenen Figuren in der Darstellung der Fig. 1b, bestückt mit einer Spritze in deren Gebrauchsanordnung;
- **Fig. 3a**: zeigt die Dosiervorrichtung der vorangegangenen Figuren in der Darstellung der Fig. 2a während des Abrüstens der Spritze aus der Dosiervorrichtung;
- **Fig. 3b**: zeigt die Dosiervorrichtung der vorangegangenen Figuren in der Darstellung der Fig. 2b während des Abrüstens der Spritze aus der Dosiervorrichtung;
- **Fig. 4**: zeigt in einer Schnittdarstellung einen Teil der erfindungsgemäßen Klemmhalterung;
- **Fig. 5**: zeigt in Vergrößerung einen Ausschnitt der Darstellung der Fig. 1b; und
- **Fig. 6**: zeigt den Schnitt der Fig. 4 in einer Draufsicht.

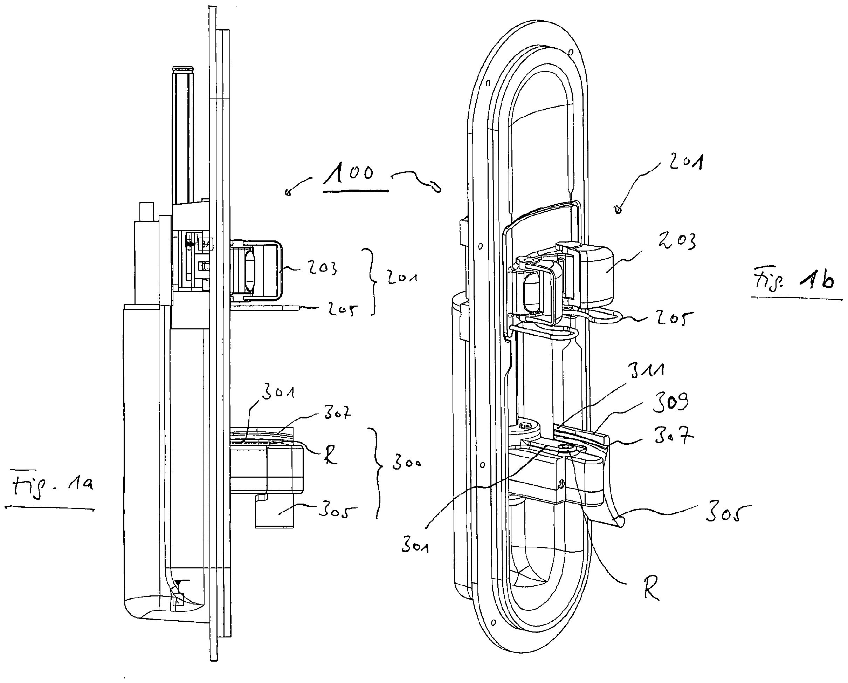

**Fig. 1a** zeigt eine erfindungsgemäße Dosiervorrichtung 100 (oder zumindest die relevantesten Komponenten hiervon) in einer Seitenansicht. **Fig. 1b** zeigt die in Fig. 1a dargestellte Dosiervorrichtung 100 in perspektivischer Ansicht. Geringfügige Maßstabunterschiede zwischen den Figuren jeweils von der Seite einerseits und in Perspektive andererseits sind möglich.

Die Dosiervorrichtung 100 weist eine (bezogen auf die Darstellung der Figur) obere Halterung 201 für eine in Fig. 1a und 1b nicht gezeigte Spritze als Beispiel einer Medikamentenabgabeeinrichtung auf. Die obere Halterung 201 weist eine Aufnahme 203 und einen Bügel 205 auf.

Die Dosiervorrichtung 100 weist zudem ein Griffstück als untere Halterung auf, ausgestaltet als erfindungsgemäße Klemmhalterung 300. Die Klemmhalterung 300 weist eine Auflage 301 auf, auf welcher die hier nicht gezeigte Spritze im Gebrauchszustand aufsitzen kann. Die Auflage 301 weist einen Abrollradius R auf, mit welchem sie an ihren schmalen Seiten oder Abschnitten hiervon kurvig abfällt. Die Kurve, in welcher sie abfällt, muss - obgleich hier so bezeichnet - selbstverständlich nicht zwingend mit der Angabe eines einzigen Radius zu beschreiben sein. Die mathematische Formulierung des geometrischen Verlaufs des Abrollradius kann aufwendiger sein.

Ein Stempel oder Spritzenstempel einer auf der Auflage 301 aufsitzenden, nicht dargestellten Spritze wird - wie in den nachfolgenden Figuren zu erkennen ist - als Beispiel für den Abschnitt der Medikamentenabgabeeinrichtung mittels wenigstens einer Klemmvorrichtung 305 gehalten oder in wenigstens einem Freiheitsgrad begrenzt.

Zwar ist in den Fig. 1a und 1b nur jeweils eine Klemmvorrichtung 305 gezeigt. Dies dient einerseits der besseren Darstellbarkeit vor allem einer Vertiefung 307. Es ist offensichtlich, dass die Klemmhalterung 300 mehr als nur eine Klemmvorrichtung 305 aufweisen kann. So kann eine zweite Klemmvorrichtung vorgesehen sein, welche der gezeigten Klemmvorrichtung 305 gegenüberliegt. Zudem kann in bestimmten erfindungsgemäßen Ausführungsformen eine dritte (sowie wiederum weitere Klemmvorrichtungen) vorgesehen sein. Andererseits dient die Darstellung von nur einer Klemmvorrichtung 305, wie in Fig. 1a oder 1b, dem Fachmann zu erkennen, dass die erfindungsgemäße Klemmhalterung 300 in manchen erfindungsgemäßen Ausführungsformen durchaus auch mit nur einer Klemmvorrichtung 305 verwendet werden kann. In derartigen Ausführungsformen kann ein begrenzender Anschlag, eine Stufe, ein Gegenlager oder dergleichen vorgesehen sein, mit welcher bzw. mit welchem zusammen die Klemmwirkung der vorhandenen einen Klemmvorrichtung 305 z. B. auf den Stempel einer Spritze erzeugt werden kann.

Gut zu erkennen ist ferner die als Nut ausgestaltete Vertiefung 307. Die Vertiefung 307 ist innerhalb des Materials bzw. der Dicke der Klemmvorrichtung 305 vorgesehen. Ist mehr als nur eine Klemmvorrichtung 305 vorgesehen, so kann eine solche Vertiefung in nur einer, in jeder oder in manchen der Klemmvorrichtungen ausgestaltet sein.

Die Vertiefung 307 verläuft in einer horizontalen Richtung der Dosiervorrichtung 100 der Fig. 1a und 1b (d. h. in einer Richtung von rechts nach links in diesen beiden Figuren) in einem Bogen, nicht aber gerade. Der Bogen ist bezogen auf die Fig. 1a und 1b nach oben konvex, nach unten konkav. Die Vertiefung 307 verläuft in der Fig. 1a erkennbar über die gesamte Tiefe der Klemmvorrichtung 305 (d. h. von links nach rechts bezogen auf die Darstellung der Fig. la). Die Vertiefung 307 kann sich in anderen Ausführungsformen allerdings auch über nur einen Teil der Tiefe der Klemmvorrichtung 305 erstrecken.

Wenig gut - und an sich nur in Fig. 1b, nicht aber auch wirklich in Fig. 1a - zu erkennen ist ein kurvenförmiger oder bogenförmiger Verlauf 311 einer Stirnfläche 309 der Klemmvorrichtung 305. Der kurvenförmige Verlauf der Stirnfläche 309 gibt einem Abschnitt der Klemmvorrichtung 305 in einer Draufsicht auf die Stirnfläche 309 - ebenfalls - einen bogenförmigen Verlauf. Der zu erkennende Bogen macht die Stirnfläche 309 in einer Richtung in die Dosiervorrichtung 100 hinein schmaler; in Abschnitten, welche nicht bogenförmig verlaufen, ist die Stirnfläche 309 breiter. Der hier beschriebene bogenförmige Verlauf 311 ist unabhängig vom bogenförmigen Verlauf der Vertiefung 307.

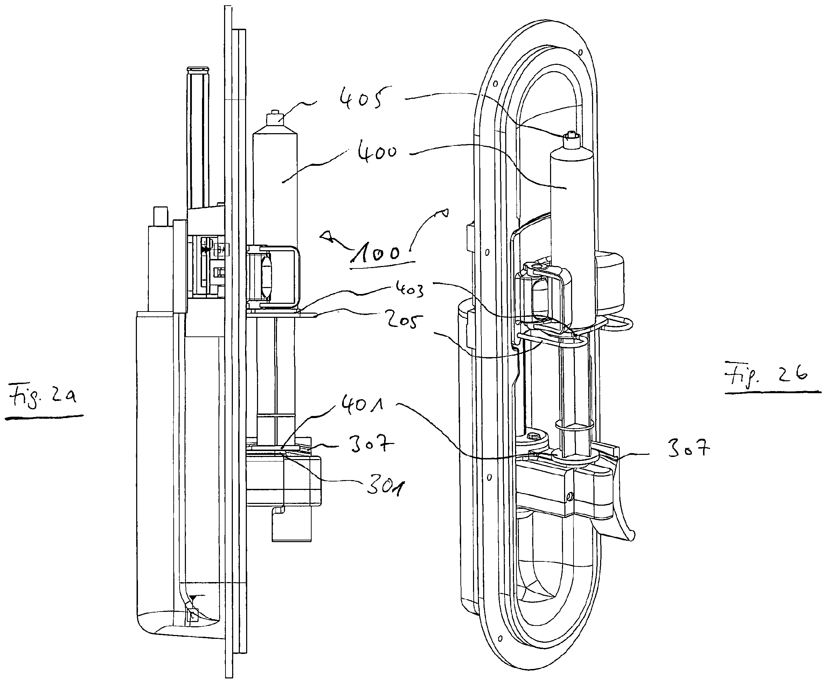

Die **Fig. 2a und 2b** zeigen die Dosiervorrichtung 100 der Fig. 1a und 1b. Die Fig. 2a und 2b unterscheiden sich von den Darstellungen der Fig. 1a und 1b allein darin, dass zusätzlich eine Spritze 400 als Beispiel einer Medikamentenabgabeeinrichtung gezeigt ist. Die Spritze 400 weist einen Spritzenstempel 401 (oder kurz: Stempel 401) auf, welcher in dem in den Fig. 2a und 2b gezeigten Gebrauchszustand der Spritze 400 bzw. der Dosiervorrichtung 100 auf der Auflage 301 aufliegt oder aufsitzt. Mit einem weiteren Abschnitt 403 der Spritze 400 liegt diese ferner auf dem Bügel 205 auf. Die Spritze 400 weist eine Düse oder Öffnung 405 auf.

Die Vertiefung 307 erlaubt erkennbar ein Kippen oder Verdrehen der Spritze 400 wenn diese in die Dosiervorrichtung 100 eingelegt wird oder aus dieser entnommen wird.

Mit der beginnenden Drehbewegung der Spritze 400 wird der Stempel 401 gegen die Auflage 301 mit dem Abrollradius R gedrückt. Kippt der Stempel 401 bzw. die Spritze 400 weiter um ihre Achse, so drückt die Außenkante oder der Umfang des Stempels 401 über den bogenförmigen Verlauf der Stirnfläche 309 die Klemmvorrichtung 305 entgegen der Federwirkung in eine Öffnungsstellung.

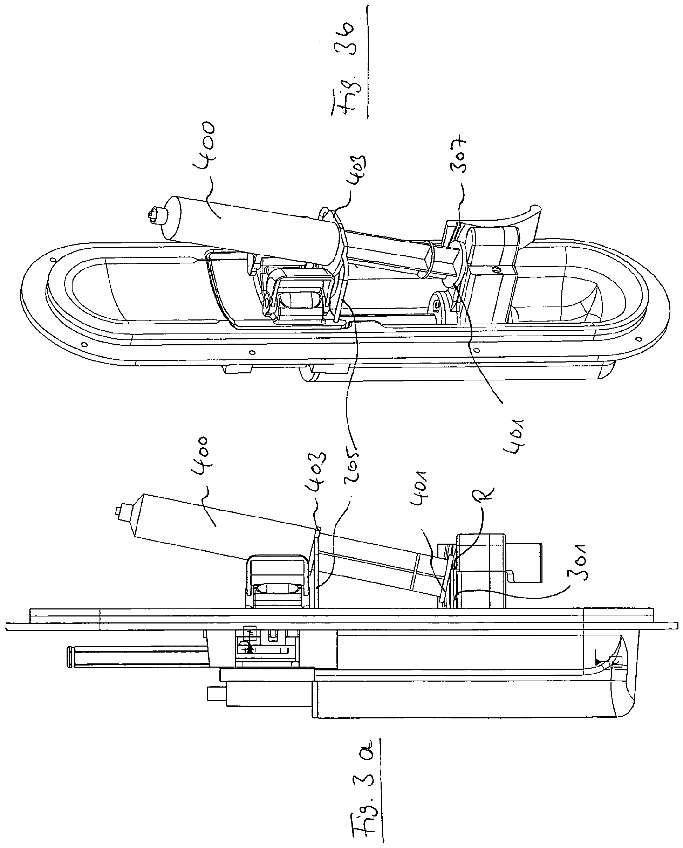

Die **Fig. 3a und 3b** zeigen die Dosiervorrichtung 100 der vorangegangenen Figuren in der Darstellung der Fig. 2a und 2b, jeweils während des Abrüstens der Spritze 400 aus der Dosiervorrichtung 100.

Gut zu erkennen ist, dass der Stempel 401 der Spritze 400 über den Abrollradius R der Auflage 301 abrollen oder sanft gekippt werden kann.

Zu erkennen ist zudem, dass der Stempel 401 mit seinem Umfang bei seinem Kippen in die bogenförmige Aussparung in der Stirnfläche 309 einfahren kann. Der Verlauf der bogenförmigen Aussparung wird dem sich beim Kippen mit unterschiedlichem Durchmesser in die Höhe der Aussparung begebenden Stempel gerecht und kann für einen sanften Anstieg sorgen, mit welchem die Klemmvorrichtung 305 gegen eine Federwirkung nach außen gedrückt wird.

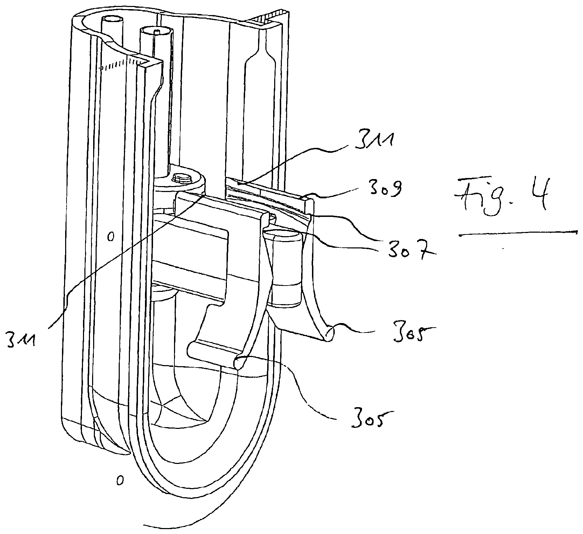

**Fig. 4** zeigt in einer Schnittdarstellung einen Teil der erfindungsgemäßen Klemmhalterung 300. Diese hat in Fig. 4 zwei sich gegenüberliegende Klemmvorrichtungen 305 mit je einer bogenartig verlaufenden Vertiefung 307. Die Vertiefungen 307 verlaufen jeweils unterhalb jeweils der Stirnfläche 309. Der bogenförmige Verlauf 311 der Stirnfläche 309 ist an beiden Klemmvorrichtungen 305 zu erkennen.

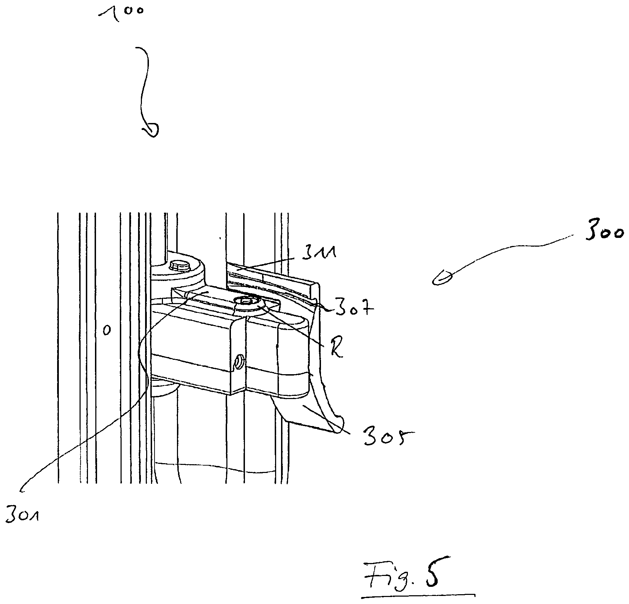

**Fig. 5** zeigt in Vergrößerung einen Ausschnitt der Darstellung der Fig. 1b.

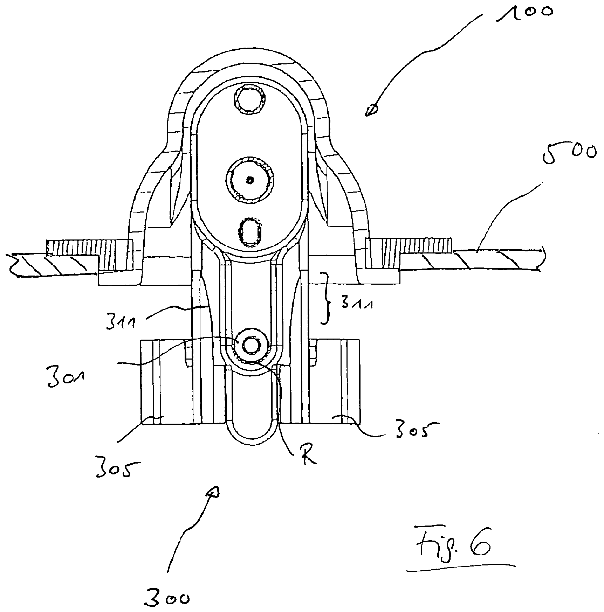

**Fig. 6** zeigt den Schnitt der Fig. 4 in einer Draufsicht. Gut zu erkennen ist der bogenförmige Verlauf 311 der Stirnfläche 309 bei beiden Klemmvorrichtungen 305.

Ergänzend zu dem bereits in Fig. 4 Dargestellten ist in Fig. 6 ein Abschnitt eines Gehäuses einer erfindungsgemäßen Blutbehandlungsvorrichtung 500 im Querschnitt gezeigt.

### Bezugszeichenliste

- 100: Dosiervorrichtung
- 201: obere Halterung
- 203: Aufnahme
- 205: Bügel
- 300: Klemmhalterung
- 301: Auflage
- 305: Klemmvorrichtung
- 307: Vertiefung
- 309: Stirnfläche
- 311: bogenförmiger Verlauf der Stirnfläche 309
- 400: Spritze
- 401: Stempel
- 403: Abschnitt der Spritze 400
- 405: Düse oder Öffnung der Spritze 400
- 500: Blutbehandlungsvorrichtung
- R: Abrollradius der Auflage 301

## Patentansprüche

1. Klemmhalterung (300) zur Aufnahme wenigstens eines Abschnitts einer Medikamentenabgabeeinrichtung oder zu dessen Begrenzung in wenigstens einer Bewegungsrichtung, wobei die Medikamentenabgabeeinrichtung eine Aufnahmekammer für die abzugebende Medikamentenlösung oder Substanz aufweist, wobei die Klemmhalterung (300) wenigstens eine Klemmvorrichtung (305) aufweist, wobei die Klemmvorrichtung (305) wenigstens eine Vertiefung (307) aufweist, welche vorgesehen ist zur Aufnahme oder Begrenzung wenigstens eines Teils des Abschnitts der Medikamentenabgabeeinrichtung, wobei
die Vertiefung (307) oder wenigstens ein Abschnitt hiervon eine bogenförmig oder kurvenförmig verlaufende Abgrenzung gegenüber nicht vertieften Abschnitten der Klemmvorrichtung (305) aufweist, und/oder wobei die Vertiefung (307) oder wenigstens ein Abschnitt hiervon in wenigstens einem Abschnitt hiervon bogenförmig oder kurvenförmig verläuft,
**dadurch gekennzeichnet, dass**
die Vertiefung (307) als Nut ausgestaltet ist und in einer Richtung senkrecht zu einer Verschieberichtung des Abschnitts der Medikamentenabgabeeinrichtung und/oder in einer Ebene parallel zu der Verschieberichtung des Abschnitts der Medikamentenabgabeeinrichtung in einem Bogen verläuft, der bezogen auf die Verschieberichtung in Richtung zu einer Düse der Medikamentenabgabeeinrichtung nach außen gewölbt oder konvex, in der entgegengesetzten Richtung nach innen gewölbt oder konkav ist.

2. Klemmhalterung (300) nach Anspruch 1, wobei die Medikamentenabgabeeinrichtung eine Spritze (400) ist, und wobei der Abschnitt der Medikamentenabgabeeinrichtung ein in der Spritze (400) zum Zwecke der Abgabe des Medikaments verschiebbarer Stempel (401) ist.

3. Klemmhalterung (300) nach Anspruch 1 und/oder 2, welche eine Auflage (301) aufweist, auf welcher die Medikamentenabgabeeinrichtung in ihrem Gebrauchszustand, insbesondere mit ihrem mittels der Klemmvorrichtung (305) in seiner Bewegung beschränkten Abschnitt, ruht, wobei die Auflage (301) einen Abrollradius (R) aufweist, unter welchem die Auflage (301) an wenigstens einem Übergang einer Auflagefläche zu einer Stirnseite oder Seitenfläche abgerundet ist.

4. Klemmhalterung (300) nach wenigstens einem der Ansprüche 1 bis 3, wobei die Klemmvorrichtung (305) wenigstens einen Stirnbereich oder eine Stirnfläche (309) aufweist, welche(r) einen kurven- oder bogenförmigen Verlauf (311) oder eine kurven oder bogenförmig begrenzte Fläche aufweist.

5. Klemmhalterung (300) nach wenigstens einem der vorangegangenen Ansprüche, wobei die Klemmvorrichtung (305) eine Feder aufweist oder hiermit verbunden ist.

6. Klemmhalterung (300) nach wenigstens einem der vorangegangenen Ansprüche, wobei die Klemmhalterung (300) zwei Klemmvorrichtungen (305) aufweist zur Aufnahme des Abschnitts.

7. Klemmhalterung (300) nach wenigstens einem der vorangegangenen Ansprüche, wobei die Klemmvorrichtung (305) ein elastisches Material aufweist, aus diesem besteht oder mit einem solchen verbunden ist.

8. Klemmhalterung (300) nach wenigstens einem der vorangegangenen Ansprüche, wobei die Vertiefung (307) derart innerhalb oder an der Klemmvorrichtung (305) vorgesehen ist, dass ein erster Querschnitt durch die Klemmvorrichtung (305) oberhalb der Vertiefung (307) dieselbe Form hat wie ein zweiter Querschnitt durch die Klemmvorrichtung (305) unterhalb der Vertiefung (307).

9. Klemmhalterung (300) nach wenigstens einem der vorangegangenen Ansprüche, wobei die Vertiefung (307) in einer Richtung senkrecht zu einer Verschieberichtung des Abschnitts der Medikamentenabgabeeinrichtung über die gesamte Tiefe der Klemmvorrichtung (305) in einem Bogen verläuft.

10. Dosiervorrichtung (100) zum Dosieren medizinischer Lösungen oder Substanzen, welche wenigstens eine Klemmhalterung (300) nach wenigstens einem der Ansprüche 1 bis 9 aufweist.

11. Dosiervorrichtung (100) nach Anspruch 10 in dessen Rückbezug auf wenigstens einen der Ansprüche 4 bis 9, wobei der kurvenförmige oder bogenförmige Verlauf der Stirnfläche (309) die Stirnfläche (309) in einer Richtung in die Dosiervorrichtung (100) hinein schmaler, in Abschnitten, welche nicht bogenförmig verlaufen, die Stirnfläche (309) breiter macht.

12. Blutbehandlungsvorrichtung (500) zur extrakorporalen Behandlung von Blut eines Patienten, welche wenigstens eine Klemmhalterung (300) gemäß wenigstens einem der Ansprüche 1 bis 9 oder eine Dosiervorrichtung (100) gemäß wenigstens einem der Ansprüche 10 bis 11 aufweist.

13. Blutbehandlungsvorrichtung (500) nach Anspruch 12, ausgestaltet als Akutdialysemaschine.

14. Blutbehandlungsvorrichtung (500) nach Anspruch 12 oder 13, ausgestaltet als Dialysevorrichtung, insbesondere als Hämodialysevorrichtung, Hämofiltrationsvorrichtung, Hämodiafiltrationsvorrichtung oder dergleichen.

## Claims

1. A clamp mounting (300) for accommodating at least one section of a drug delivery device or for restricting it in at least one direction of motion, wherein the drug delivery device comprises an accommodation chamber for drug solution or substance to be delivered, wherein the clamp mounting (300) comprises at least one clamping device (305), wherein the clamping device (305) comprises at least one recess (307) which is provided for accommodating or restricting at least one part of the section of the drug delivery device,
wherein the recess (307) or at least a section hereof comprises an arched or curved demarcation between itself and sections of the clamping device (305) that are not recessed, and/or wherein the recess (307) or at least one section hereof in at least one section hereof is arch-shaped or curve-shaped,
**characterized in that**
the recess (307) is configured as a groove and has an arched course in a direction perpendicular to a shifting direction of the section of the drug delivery device and/or in a plane parallel to the shifting direction of the section of the drug delivery device runs in a curve, that is outwardly curved or convex in relation to the shifting direction in direction of a nozzle of the drug delivery device, in the opposed direction inwards curved or concave.

2. The clamp mounting (300) according to claim 1, wherein the drug delivery device is a syringe (400), and wherein the section of the drug delivery device is a piston (401) which is movable within the syringe (400) for the purpose of delivery of the drug.

3. The clamp mounting (300) according to claim 1 and/or 2, comprising a support (301) on which the drug delivery device, in its state of use, rests, in particular with its section which is limited in its motion by means of the clamping device (305), wherein the support (301) has a rolling radius (R), under which the support (301) is rounded at at least one transition of a supporting surface to a front surface or lateral surface.

4. The clamp mounting (300) according to at least one of the claims 1 to 3, wherein the clamping device (305) comprises at least one front area or front surface (309), which has a curved or arched (311) course or an area that is delimited in curve- or arch-shape.

5. The clamp mounting (300) according at least one of the preceding claims, wherein the clamping device (305) comprises a spring or is connected hereto.

6. The clamp mounting (300) according to at least one of the preceding claims, wherein the clamp mounting (300) comprises two clamping devices (305) for accommodating the section.

7. The clamp mounting (300) according to at least one of the preceding claims, wherein the clamping device (305) comprises an elastic material, consists of this or is connected with such one.

8. The clamp mounting (300) according to at least one of the preceding claims, wherein the recess (307) is provided within or at the clamping device (305), such that a first cross section through the clamping device (305) above the recess (307) has the same form as a second cross section through the clamping device (305) below the recess (307).

9. The clamp mounting (300) according to at least one of the preceding claims, wherein the recess (307) has an arched course in a direction perpendicular to a shifting direction of the section of the drug delivery device along the whole depth of the clamping device (305).

10. A dosing apparatus (100), for dosing medical solutions or substances, comprising at least one clamp mounting (300) according to at least one of the claims 1 to 9.

11. The dosing apparatus (100), according to the claim 10 referring to at least one of the claims 4 to 9, wherein the curved or arched course of the front surface (309) narrows the front surface (309) in a direction to an interior of the dosing apparatus (100), and widens the front surface (309) in non-arched sections.

12. A blood treatment apparatus (500) for the extracorporeal treatment of blood of a patient, comprising at least one clamp mounting (300) according to at least one of the claims 1 to 9 or a dosing apparatus (100) according to at least one of the claims 10 to 11.

13. The blood treatment apparatus (500) according to claim 12, embodied as an acute dialysis machine.

14. The blood treatment apparatus (500) according to claim 12 or 13, embodied as a dialysis apparatus, in particular as a hemodialysis apparatus, hemofiltration apparatus, hemodiafiltration apparatus or the like.

## Revendications

1. Un dispositif de retenue par serrage (300) destiné à recevoir au moins une section d'un dispositif d'administration de médicaments ou à sa restriction dans au moins une direction de mouvement, où le dispositif d'administration de médicaments présente une chambre de réception pour la solution ou la substance médicamenteuse devant être administrée, où le dispositif de retenue par serrage (300) présente au moins un dispositif de serrage (305), où le dispositif de serrage (305) présente au moins une cavité (307) prévue pour recevoir ou restreindre au moins une partie de la section du dispositif d'administration de médicaments,
où la cavité (307), ou au moins une section de celle-ci, présente une délimitation, s' étend en forme d'arc ou en forme de courbe par rapport aux parties non évidées du dispositif de serrage (305), et/ou où la cavité (307), ou au moins une section de celle-ci, s'étend sous la forme d'un arc ou sous la forme d'une courbe dans au moins une partie de celle-ci,
**caractérisé en ce que**
la cavité (307) est configurée en tant que rainure et s'étend en arc perpendiculairement à une direction de déplacement de la section du dispositif d'administration de médicaments et/ou dans un plan parallèlement à la direction de déplacement de la section du dispositif d'administration de médicaments, l'arc étant relativement à la direction de déplacement bombé vers l'extérieur ou convexe en direction d'une buse du dispositif d'administration de médicaments, dans la direction opposée bombé vers l'intérieur ou concave.

2. Le dispositif de retenue par serrage (300) selon la première revendication, où le dispositif d'administration de médicaments est une seringue (400) et où la section du dispositif d'administration de médicaments est un piston mobile (401) à l'intérieur de la seringue (400) permettant l'administration du médicament.

3. Le dispositif de retenue par serrage (300) selon la revendication 1 et/ou 2, qui comprend un support (301), sur lequel repose le dispositif d'administration de médicaments dans son état d'utilisation, en particulier au moyen de sa section limitée en mouvement grâce au dispositif de serrage (305), où le support (301) comprend un rayon de roulement (R), sous lequel le support (301) est arrondi au moins à la transition d'une surface d'appui à une face frontale ou une surface latérale.

4. Le dispositif de retenue par serrage (300) selon au moins l'une quelconque des revendications 1 à 3, où le dispositif de serrage (305) présente au moins une zone frontale ou une surface frontale (309), présentant un tracé (311) de forme courbée ou arquée ou présentant une surface limitée par une forme courbée ou arquée.

5. Le dispositif de retenue par serrage (300) selon au moins l'une quelconque des revendications précédentes, où le dispositif de serrage (305) présente un ressort ou est connecté avec celui-ci.

6. Le dispositif de retenue par serrage (300) selon au moins l'une quelconque des revendications précédentes, où le dispositif de retenue par serrage (300) présente deux dispositifs de serrage (305) destinés à recevoir la section.

7. Le dispositif de retenue par serrage (300) selon au moins l'une quelconque des revendications précédentes, où le dispositif de serrage (305) présente un matériau élastique, en est constitué ou est connecté avec un tel matérieau.

8. Le dispositif de retenue par serrage (300) selon au moins l'une quelconque des revendications précédentes, où la cavité (307) est prévue à l'intérieur ou sur le dispositif de serrage (305), de telle sorte qu'une première coupe transversale du dispositif de serrage (305) au-dessus de la cavité (307) ait la même forme qu'une seconde coupe transversale du dispositif de serrage (305) au-dessous de la cavité (307).

9. Le dispositif de retenue par serrage (300) selon au moins l'une quelconque des revendications précédentes, où la cavité (307) s'étend sous la forme d'un arc sur toute la profondeur du dispositif de serrage (305) perpendiculairement à la direction de déplacement de la section du dispositif d'administration de médicaments.

10. Un dispositif de dosage (100), destiné à doser des solutions médicamenteuses ou des substances, présentant au moins un dispositif de retenue par serrage (300) selon au moins l'une quelconque des revendications 1 à 9.

11. Le dispositif de dosage (100) selon la revendication 10 qui dépend au moins d'une des revendications 4 à 9, où le tracé de forme courbée ou arquée de la surface frontale (309) rétrécit la surface frontale (309) dans une direction orientée vers l'intérieur du dispositif de dosage (100), et élargit la surface frontale (309) dans les sections ne s'étendant pas sous la forme d'une courbe.

12. Un dispositif de traitement du sang (500) destiné au traitement extracorporel du sang d'un patient, comprenant au moins un dispositif de retenue par serrage (300) selon l'une quelconque des revendications 1 à 9 ou un dispositif de dosage (100) selon au moins l'une quelconque des revendications 10 à 11.

13. Le dispositif de traitement du sang (500) selon la revendication 12, conçu en tant que machine aiguë de dialyse aiguë.

14. Le dispositif de traitement du sang (500) selon la revendication 12 ou 13, conçu en tant qu' appareil de dialyse, en particulier en tant qu'appareil d'hémodialyse, qu'appareil d'hémofiltration, qu'appareil d'hémodiafiltration ou similaire.
